# EUROPEAN PATENT APPLICATION

(11) **EP 2 954 919 A1**
(43) Date of publication of application: **16.12.2015**
(21) Application number: 14305879.0
(22) Date of filing: 11.06.2014
(51) Int. Cl.: A61M 16/06, A61M 16/08, A61M 16/00

(54) **Respiratory mask with venting holes**

(71) Applicant: Air Liquide Medical Systems, 92160 Antony (FR)
(72) Inventor: Masserdotti, Fulvio, 25075 BRESCIA (IT); Alberici, Luca, 25128 BRESCIA (IT); Sandoni, Giuseppe, 25124 BRESCIA (IT)
(74) Representative: Pittis, Olivier

(57) **Abstract**

A respiratory mask (20), preferably a nasal mask, comprising a hollow body (1) comprising an inner chamber (2) and a gas inlet (3) in fluid communication with said inner chamber (2), said gas inlet (3) comprising an annular peripheral border (4) comprising a connecting structure (6, 7) for fixing thereto a tubular gas connector (10). Several venting ports (5) are arranged in said annular peripheral border (4) so that the inner chamber (2) is in fluid communication with the ambient atmosphere through said venting ports (5). The respiratory mask of the present invention is useable for treatment of a respiratory disorder or condition in non-invasive positive pressure ventilation or in a nasal continuous positive airway pressure therapy of sleep disordered breathing conditions, such as obstructive sleep apnea.

## Description

The invention concerns a respiratory mask, in particular a nasal mask, with improved venting ports used for venting CO2-rich gases to the atmosphere, while the patient is expiring gases, in particular for adult or pediatric uses in the treatment of respiratory conditions or diseases affecting adults, children, toddlers, infants or newborns.

Nasal masks are commonly used for delivering non-invasive positive pressure ventilation (NPPV) or for nasal continuous positive airway pressure (N-CPAP) therapy in disordered breathing conditions, such as obstructive sleep apnea (OSA), chronic obstructive pulmonary disease (COPD).

Nasal masks deliver a flow of breathable gas for or to assist in patient respiration.

Such a mask assembly typically comprises a rigid or semi-rigid hollow shell, usually made of polymer, defining a breathing chamber that receives at least a part of the patient's nose and further comprising a soft face-contacting cushion that comes into contact with the patient's face and conforms to the various facial contours of the patient face. The cushion is usually made of soft, resilient elastomeric material, such as soft silicone or similar, and a forehead support and a headgear for correctly positioning, maintaining and/or securing the mask on the head of a patient.

The forehead support is usually arranged on an expansion part of the mask, i.e. a portion of the mask body forming a holding arm that projects upwardly from the mask body and in the direction of the forehead of the user when the mask is positioned on the user's face, which holding arm is also usually made of polymer. The hollow shell typically receives a gas supply line which delivers a respiratory gas, such as air under pressure, into the breathing chamber of the shell. An example of a nasal mask of this kind is given by EP-A-462701.

The mask assembly is secured to the wearer's head by straps or similar devices thereby forming a headgear that can be adjusted to pull the mask against the face with sufficient force to achieve a gas tight seal between the mask and the wearer's face as taught by EP-A-462701, EP-A-874667, EP-A-1972357 or WO-A-00/57942.

During expiration phases, the CO₂-enriched gas flow exhaled by the patient should be vented to the atmosphere for avoiding or limit CO₂ re-inhalation by the patient during the inhalation phases.

To this end, it is common to arrange one or several venting ports either in the mask shell, as for example taught by EP-A-1582230, or in the wall of the tubular connector connected to the mask shell, as taught for example by WO-A-2005/051468 or EP-A-2281597.

However, there is still a need for improving the venting to the atmosphere of the CO₂-enriched gas exhaled by the patient.

Hence, the problem to be solved is to provide an improved respiratory mask architecture, especially a nasal mask, that allows an efficient CO₂-gas venting to the atmosphere.

The solution of the present invention concerns a respiratory mask, in particular a nasal mask, comprising a hollow body comprising an inner chamber and a gas inlet in fluid communication with said inner chamber, said gas inlet comprising an annular peripheral border comprising a connecting structure for fixing thereto a tubular gas connector, characterized in that several venting ports are arranged in said annular peripheral border so that the inner chamber is in fluid communication with the ambient atmosphere through said venting ports.

The mask according to the present invention can further comprise one or more of the following additional features:
- venting ports, which are orifices that allow the venting of gas to the atmosphere.
- the connecting structure comprises an outer annular wall and an inner annular wall arranged face to face.
- venting ports are arranged in the outer annular wall and/or in the inner annular wall.
- the outer annular wall and the inner annular wall are linked by a linking wall forming the front surface of the border.
- the mask comprises at least 10 venting ports.
- the venting ports are distributed all along the circumference of the outer annular wall and/or the inner annular wall.
- the mask comprises a tubular gas connector inserted into the gas inlet.
- the tubular gas connector comprises a front tubular portion cooperating with the inner annular wall so as to maintain the tubular gas connector integral with the mask body.
- the inner annular wall comprises an annular shoulder and the tubular gas connector comprises an peripheral abutment, said peripheral abutment bearing on the annular shoulder, when the tubular gas connector is inserted into the gas inlet.
- a spacing is arranged between the outer annular wall and the inner annular wall.
- when the tubular gas connector is inserted into the gas inlet, a part of the peripheral outer surface of said tubular gas connector delimits with the inner annular wall a gas passage, said gas passage being in fluid communication with venting holes arranged in the inner annular wall, and with the ambient atmosphere.
- the mask further comprises a holding arm projecting upwardly from the hollow body, and two lateral arms projecting laterally from the hollow body, the two lateral arms and the holding arm being integrally fixed to said hollow body, preferably a headgear comprising several straps is fixed to the two lateral arms and the holding arm.
- the mask further comprises a flexible cushion fixed to the hollow body and comprising a respiratory chamber with an aperture adapted for receiving at least part of the patient's nose, when the patient wears the mask, preferably the flexible cushion comprises one or several membranes.
- the hollow body has a tubular-shape portion.
- the hollow body is constituted by a ring- or tube- element or piece forming a tubular-shape portion.
- the hollow body has a generally cylindrical form, tronconical form or a combination thereof.
- the two lateral arms and the holding arm are integral with the peripheral wall of said the hollow body.
- the hollow body, the holding arm and of the two lateral arms are molded in one piece.
- the hollow body, the holding arm and of the two lateral arms are made of a flexible material.
- the hollow body, the holding arm and of the two lateral arms are made of a polymer material, such as polycarbonate (PC), polypropylene (PP) or nylon.
- the two lateral arms and the holding arm each comprises a proximal end integral with the hollow body, and a free distal end.
- the hollow body is traversed by a central passage for conveying a gas, said central passage comprising an inlet orifice and an outlet orifice having each a diameter of between 1 and 2.5 cm.
- the tubular-shape portion of the hollow body has a length of between 0.5 and 3cm.
- the holding arm and of the two lateral arms have an elongated shape.
- the holding arm and of the two lateral arms have for example a band or ribbon shape. Other shapes are possible.
- the holding arm has a length of about 2 and 8 cm.
- each lateral arms has a length of about 3 and 6 cm.
- the free distal ends of the holding arm and of the two lateral arms comprise a strap-fixing system for fixing thereto the straps of a headgear.
- the hollow body, the holding arm and of the two lateral arms are made of a flexible polymeric material so as to be slightly bendable toward the patient's face under the forces applied by the headgear, when said headgear is adjusted to the morphology of the patient's head.
- the strap-fixing system carried by the free distal ends of the holding arm and of the two lateral arms comprise at least one slot and/or fixing hook for fixing straps of a headgear.
- the strap-fixing system carried by the free distal end of the holding arm comprises at least one slot, preferably two slots, such as open slots. For instance, the slots can have a width of between 8 to 35 mm.
- the strap-fixing system carried by each free distal ends of the two lateral arms comprises a fixing hook.
- the flexible cushion comprises a respiratory chamber, i.e. an inner chamber, with an aperture (i.e. a nose aperture) adapted for receiving at least part of the patient's nose, when the patient wears the mask, and an inlet orifice, said respiratory chamber being in fluid communication with the central passage of the tubular portion of the hollow body through said inlet orifice.
- the flexible cushion further comprises one or several membranes, preferably two superimposed membranes that ensure an efficient gas tightness while being in contact with the patient's face, when the patient wears the mask.
- the flexible cushion further comprises one or several membranes arranged around along or part of the periphery of the aperture of the respiratory chamber. In other words, the membrane(s) form(s) a flexible skirt around the along or part of the periphery of the aperture of the respiratory chamber.
- at least a region of the holding arm and of the two lateral arms is configured or shaped so as to spouse, i.e. match, the external profile, i.e. the outer contour, of the flexible cushion.
- the holding arm and of the two lateral arms is configured to have a curved-shape.
- the mask further comprises a hollow curved connector fixed to the front side of the hollow body so as to be in fluid communication with one another thereby allowing a gas to circulate from the hollow curved connector to the central passage of the hollow body.
- the hollow curved connector is fixed to and rotatable with respect to the tubular-shape portion of the main body.
- the hollow curved connector has general "L" shape.
- the mask further comprises a headgear comprising several straps connected to the free distal ends of the holding arm and of the two lateral arms. Said headgear is used for maintaining and securing the mask in a desired position on the head of the patient.
- the mask further comprises a headgear comprising several straps made of polymer material or fabric material, or both.
- the flexible cushion comprises first connecting structures arranged around its inlet orifice, and the rear side of the tubular-shape portion of the hollow body comprises second connecting structures, said first connecting structures cooperating with said second connecting structures so as to fix and maintain said flexible cushion integral with the hollow body.
- said first connecting structures comprise male/female connection elements, such as grooves, walls, abutments...
- the cushion is made of a soft flexible material.
- the cushion is made of silicone or similar.
- the cushion comprises an internal chamber and an inlet orifice in fluid communication with the internal chamber.
- the membrane and the cushion are integral and formed of a unique piece made of a resilient soft or semi-soft material, i.e. of silicone or similar.
- the cushion has a general triangular, trapezoidal or saddle shape.
- the inlet orifice is arranged at the center of the cushion.
- the mask is a nasal mask.
- the cushion is configured and sized for coming into contact, when the mask is worn by the user or patient, with specific regions of the user or patient's face. Preferably, those regions comprise an intermediate nose region, an upper lip region and lateral nose regions, wherein
   - the upper lip region is the area comprised between the nose and the upper lip;
   - the intermediate nose region is the area of the nose approximately located at the junction of bone and cartilage; and
   - the lateral nose regions are those located on each side of the nose.

The invention also concerns an assembly for delivering a gas to a patient comprising a gas delivery device, such as a medical ventilator, and a respiratory mask according to the present invention, preferably the gas delivery device is connected to the respiratory mask by means of a gas line, such as a flexible hose.

Embodiments of a respiratory mask, especially a nasal mask, according to the present invention are shown in the enclosed Figures, among which:
- Figure 1 represents a general view of a first embodiment of a mask according to the present invention,
- Figure 2 is an exploded view of the mask of Figure 1,
- Figure 3 is an enlarged view of the hollow body of the mask of Figures 1 and 2,
- Figure 4 is an enlarged view of a second embodiment of the hollow body for a mask according to Figures 1 and 2,
- Figure 5 is enlarged view of the hollow body of Figure 4 connected to a hollow gas connector,
- Figures 6 is enlarged view of a portion of Figure 5 showing the circulation of expired gas through the venting holes,
- Figures 7 and 8 show the mask of Figure 1 worn by a patient.

The present invention proposes a new structure of respiratory mask comprising venting ports as shown in Figures that illustrate some embodiments of a nasal mask according to the present invention.

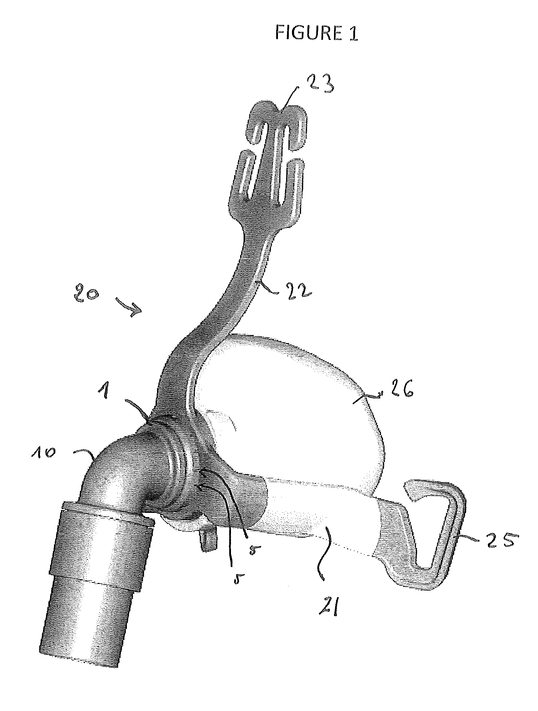

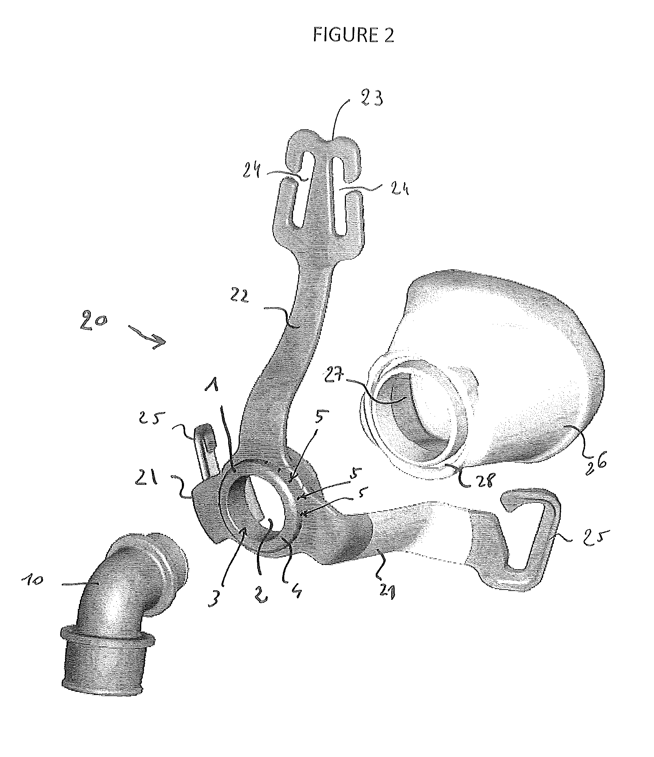

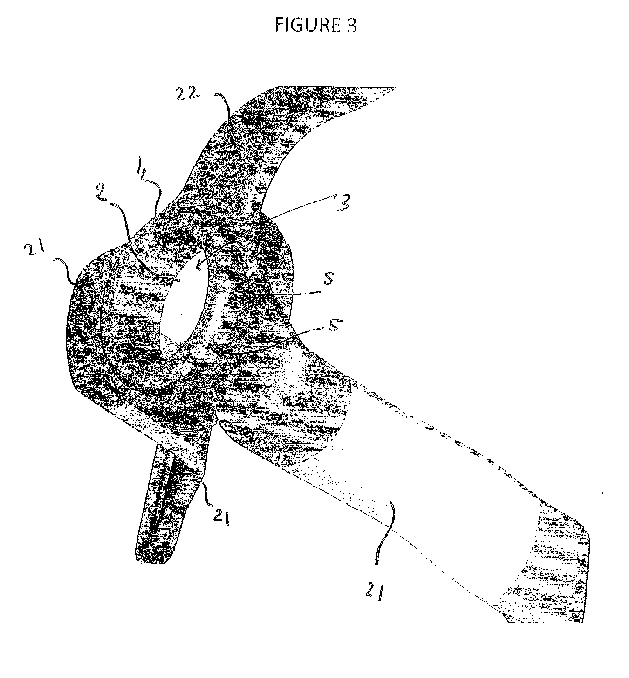

Figures 1 and 2 represent a general view of a first embodiment of a respiratory mask 20, namely a nasal mask, according to the present invention, wherein the venting holes are located as shown on Figure 3 and as detailed hereafter.

The mask 20 comprises a hollow body 1 having a particular simple structure as it is formed of a tubular-shape portion traversed by a central passage defining an inner chamber 2 and comprising a gas inlet 3 in fluid communication with said inner chamber 2, as visible in Figures 2-4. In other words, the main body 1 of the mask 20 according to the invention is essentially constituted of a hollow ring or tube element forming the tubular-shape portion and the central passage 2.

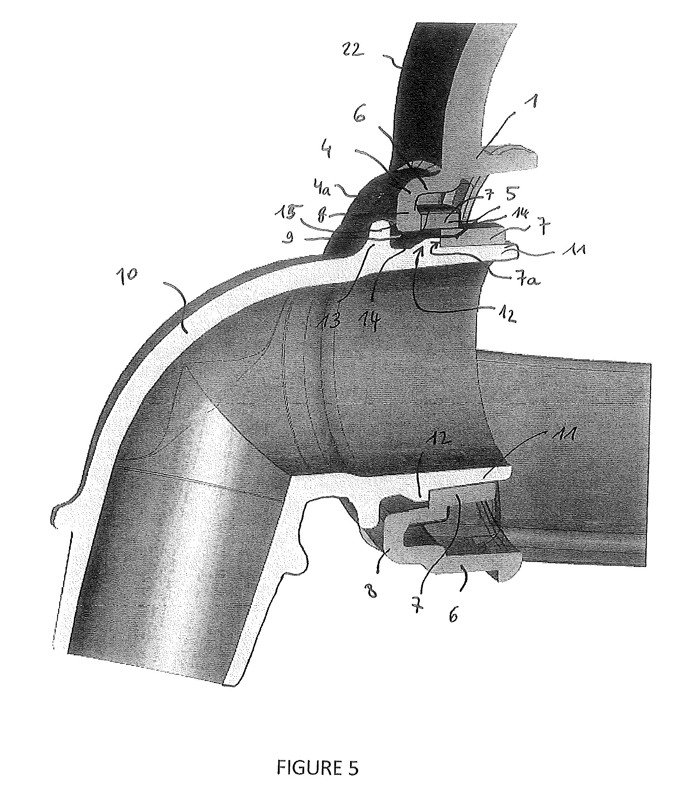

The gas inlet 3 comprises an annular peripheral border 4 comprising a connecting structure 6, 7 for fixing thereto a tubular gas connector 10 as detailed in Figure 5.

The tubular-shape portion or hollow body 1 is carrying two lateral arms 21 and a holding arm 22 that are integrally fixed to the peripheral wall or surface of said hollow body 1. The holding arm 22 projects upwardly, i.e. about vertically, from said hollow body 1, whereas the two lateral arms 21 projecting laterally from said hollow body 1 in opposite directions, i.e. one toward the right side of the mask 20 and the other toward the left side of the mask 20, as shown in Figures 1 and 2.

The two lateral arms 21 and the holding arm 22 have preferably respective lengths of between 2 and 15 cm.

Preferably, the tubular-shape portion 1, the two lateral arms 21 and the holding arm 22 are molded in one piece. Nevertheless, they can be made from several pieces or sub-units that are subsequently assembled together and made integral by means of any available fixing technique, for instance they can be glued, thermo-welded or similarly affixed together. They can be made of a polymer material that is slightly flexible, preferably a polymer or plastic material, such as polypropylene (PP), polycarbonate (PC) and nylon, or similar.

Generally speaking, the tubular-shape portion forming the hollow body can have a generally cylindrical or tronconical form or shape, or any combinations thereof. For example, the tubular-shape portion forming the hollow body 1 can be partially cylindrical and partially tronconical, or generally cylindrical but formed of sub-portions having different diameters.

Preferably, the tubular-shape portion forming the hollow body 1 has a length of between 1.5 and 8 cm and its central passage 2 has a diameter of between 1 and 4 cm.

The two lateral arms 21 and the holding arm 22 that are integrally attached to the peripheral wall or surface of said tubular-shape portion of the hollow body 1, and further comprise each a free distal end comprising a strap-fixing system 23 for fixing thereto the straps of a headgear that is used for maintaining and securing the mask 20 in a desired position on the head of the patient. Generally, the straps of headgears are made of polymer or fabric materials. In the present case, the strap-fixing system 23 carried by the free distal end of the holding arm 22 comprises an enlarged structure comprising two open slots 24, whereas the free distal ends of the two lateral arms 21 each comprise a fixing hook 25 for fixing headgear straps forming loops.

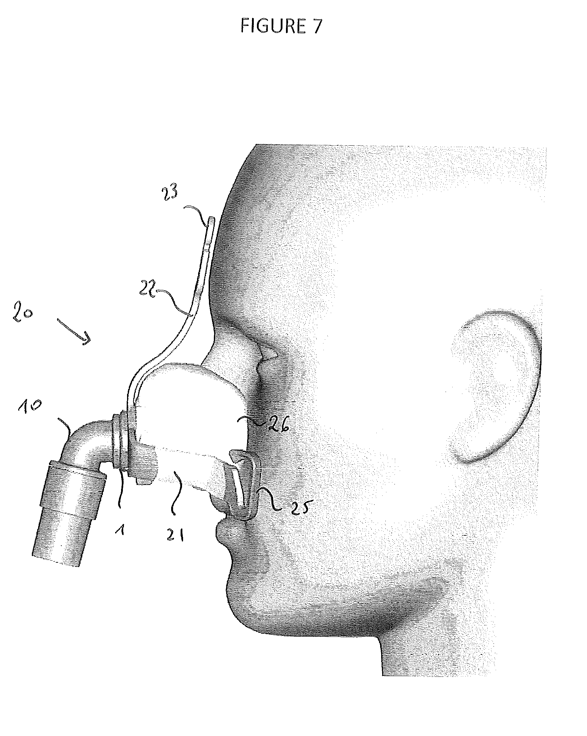

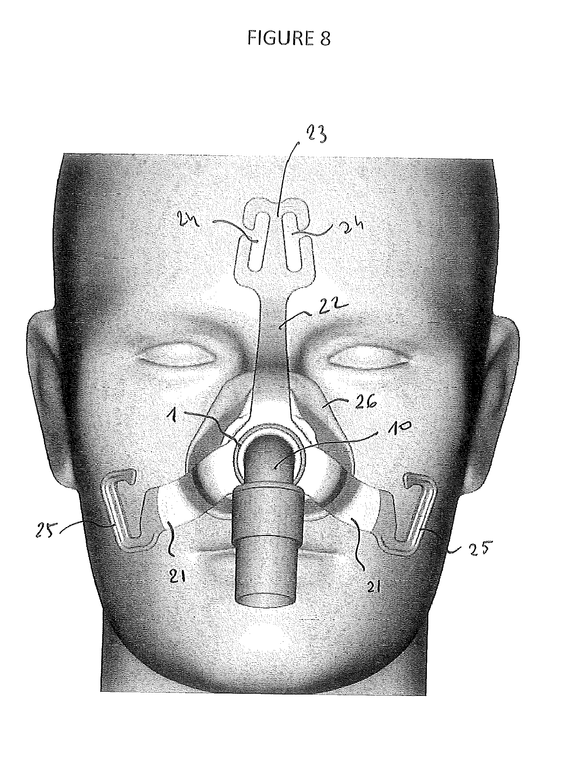

Further, the mask 20 of the present invention also comprises a flexible cushion 26 that is fixed to the rear side of the tubular-shape portion of the hollow body 1. Typically, the flexible cushion 26 is a tridimensional hollow structure forming a respiratory chamber with a nose aperture adapted, i.e. conformed and sized, for receiving at least part of the patient's nose as above explained, when the patient wears the mask, so that the patient can breathe the respiratory gas contained in said chamber as illustrated in Figures 7 and 8. The cushion body 26 further comprises an inlet orifice 27 for allowing a gas or gas mixture, such as pressurized air, coming from the hollow inner chamber 2 of the hollow body 1 to enter into the respiratory chamber, as shown in Figure 2. In other words, the respiratory chamber of the cushion 26 is in fluid communication with the central passage 2 of the tubular-shape portion of the hollow body 1 and with the lumen of the hollow curved connector 10 so that a gas flow can travel from said tubular connector 10 to said chamber of the cushion 26.

Preferably, in order to provide efficient gas tightness (i.e. seal) and/or increased comfort for the patient, the nose aperture of the cushion 26 is delimited by at least one flexible membrane, preferably two superimposed membranes, that comes into contact with the patient's face and spouses his/her facial morphology. This(those) membrane(s) constitute(s) a kind of soft flexible skirt delimiting said the nose aperture of the cushion 26. Preferably, two (or more) superimposed membranes are used as using several membranes may improve the gas tightness.

The cushion 26 has, in the present case, a generally trapezoidal or saddle tridimensional form or shape (rear view) so as to better match the contours of the patient's face, especially in the nasal regions.

When the mask is worn by the patient, i.e. when the cushion 26 receives at least a part of the patient's nose, when said patient introduces his/her nose into the internal volume of the breathing chamberland breathes the gas contained therein, the cushion 26 and the membrane(s) arranged around the peripheral border of the nose aperture are in contact with the intermediate nose region (area at the junction of the bone and cartilage of the nose), the upper lip region (area between the nose and the upper lip), and the two opposite lateral regions of the nose of the patient (i.e. right and left flange regions of the nose). Of course, other embodiments and shapes are possible.

The cushion 26 is preferably made of a flexible and soft material, such as silicone or the like. Preferably, the membrane(s) 16 and the cushion body are molded in one piece. The cushion 26 is fixed to the hollow body 1 by means of a connecting system 28, such as male/female connections, as shown in Figure 2. For example, the connecting system 28 can comprise peripheral groove(s) and wall(s) arranged on the cushion 26 cooperating with complementary peripheral wall(s) and/or groove(s) arranged on the body 1 of the mask 20 that are configured or shaped so as to perfectly fit together.

An important improvement according to the present invention concerns the venting ports 5 that are used for venting expired CO₂-rich gases to the atmosphere through those venting ports 5, when the patient exhales.

Indeed, according to the present invention, several venting ports 5 are arranged in the annular peripheral border 4 so that the inner chamber 2 of the hollow body 1 is in fluid communication with the ambient atmosphere through said venting ports 5.

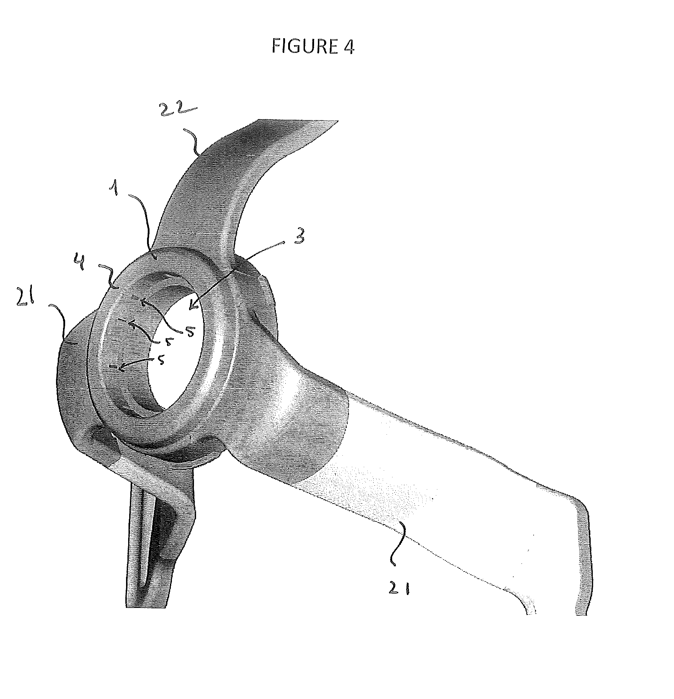

As visible in Figures 3 and 4, the annular peripheral border 4 comprising the connecting structure 6, 7 for fixing thereto the tubular gas connector 10, projects outwardly from the mask body 1.

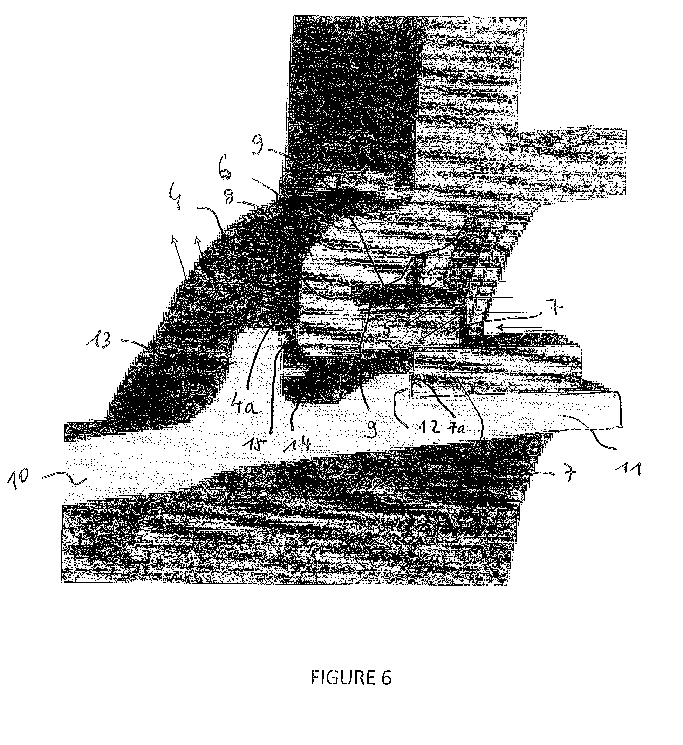

More precisely, as shown in Figures 5 and 6, said annular peripheral border 4 includes connecting structures 6, 7 that comprise an outer annular wall 6 and an inner annular wall 7 arranged face to face, and separated by an open area 9 having here a annular shape as it is delimited by said outer annular wall 6 and inner annular wall 7. In other words, the outer annular wall 6 and the inner annular wall 7 are linked by a linking wall 8 forming, here, the front surface 4a of the border 4. As visible on Figures 5 and 6, the outer annular wall 6, inner annular wall 7 and linking wall 8 form a tridimensional structure having a U-section.

Depending on the embodiment, venting ports 5 can be arranged in the outer annular wall 6 as shown in Figure 3, or in the inner annular wall 7 as shown in Figure 4, or in both of them.

Preferably, at least 10 venting ports 5 are arranged in the inner or outer walls 6, 7 of the border 4.

More preferably, the venting ports 5 are distributed all along the circumference of the outer annular wall 6 and/or the inner annular wall 7 as visible on Figures 3 and 4, or only on a part of said circumference.

Figures 5 and 6 show the tubular gas connector 10, which as a general L-shape, inserted into the gas inlet 3 of the mask hollow body 1. As one can see, the tubular gas connector 10 comprises a front tubular portion 11 cooperating with the inner annular wall 7 so as to maintain the tubular gas connector 10 integral with the mask body 1.

More precisely, the inner annular wall 7 comprises an annular shoulder 7a cooperating with a peripheral abutment 12 arranged on the peripheral surface of the tubular gas connector 10. The peripheral abutment 12 bears on the annular shoulder 7a so as to limit the course of the gas connector 10 into the gas inlet 3 and hollow body 1, when the tubular gas connector 10 is inserted into the gas inlet 3.

As illustrated in Figure 6, the CO2-rich gas (see "arrows") coming from the cushion 26, while the patient expires, enters first into the annular spacing 9 arranged between the outer annular wall 6 and the inner annular wall 7, then passes through the ports 5 and into a gas passage 14 existing between the external surface of the connector 10 and the outer surface of the annular border 4, before being vented to the atmosphere.

In this aim, as shown in Figure 5 and 6, a part of the peripheral outer surface of said tubular gas connector 10 is configured so as to delimit with the inner annular wall 7 a gas passage 14 which is in fluid communication, one the one end, with the venting holes 15 arranged in the inner annular wall 7, and, one the other hand, with the ambient atmosphere, when the tubular gas connector 10 is inserted into the gas inlet 3 of the hollow body 1.

The tubular connector 10 further comprises a deflector 13 forming a peripheral annular skirt arranged on its outer peripheral surface and that deflects the expired gas flow in a radial direction away from the mask 20. The deflector 13 is situated at the exit of the gas passage 14 and is facing the front face 4a of the annular border 4, while defining between them an annular gas exit 15 as shown in Figures 5 and 6.

Generally speaking, the respiratory mask 20 of the present invention, such as a nasal mask, is light and comfortable to wear due to a reduction of the overall size and weight of the mask, thereby allowing efficient positioning and securing of the mask 20 on the patient's face, as well as a good tightness (seal) and an improved comfort of use for the patient as shown in Figures 7 and 8.

The nasal respiratory mask 20 of the present invention can be used in a method for treatment of a respiratory disorder or condition affecting infant, toddler and child patients as well as adult patients, for example in non-invasive positive pressure ventilation (NPPV) or in a nasal continuous positive airway pressure (N-CPAP) therapy of sleep disordered breathing (SDB) conditions, such as, for example, obstructive sleep apnea (OSA).

## Claims

1. Respiratory mask (20), preferably a nasal mask, comprising a hollow body (1) comprising an inner chamber (2) and a gas inlet (3) in fluid communication with said inner chamber (2), said gas inlet (3) comprising an annular peripheral border (4) comprising a connecting structure (6, 7) for fixing thereto a tubular gas connector (10), **characterized in that** several venting ports (5) are arranged in said annular peripheral border (4) so that the inner chamber (2) is in fluid communication with the ambient atmosphere through said venting ports (5).

2. Respiratory mask according to the previous Claim, **characterized in that** at least a part of the annular peripheral border (4) comprising said connecting structure (6, 7) projects outwardly from the mask body (1).

3. Respiratory mask according to any one of the preceding Claims, **characterized in that** the connecting structure (6, 7) comprises an outer annular wall (6) and an inner annular wall (7) arranged face to face.

4. Respiratory mask according to any one of the preceding Claims, **characterized in that** venting ports (5) are arranged in the outer annular wall (6) and/or in the inner annular wall (7).

5. Respiratory mask according to any one of the preceding Claims, **characterized in that** the outer annular wall (6) and the inner annular wall (7) are linked by a linking wall (8) forming the front surface (4a) of the border (4).

6. Respiratory mask according to any one of the preceding Claims, **characterized in that** it comprises at least 10 venting ports (5).

7. Respiratory mask according to any one of the preceding Claims, **characterized in that** the venting ports (5) are distributed all along the circumference of the outer annular wall (6) and/or the inner annular wall (7).

8. Respiratory mask according to any one of the preceding Claims, **characterized in that** itcomprises a tubular gas connector (10) inserted into the gas inlet (3).

9. Respiratory mask according to any one of the preceding Claims, **characterized in that** the tubular gas connector (10) comprises a front tubular portion (11) cooperating with the inner annular wall (7) so as to maintain the tubular gas connector (10) integral with the mask body (1).

10. Respiratory mask according to any one of the preceding Claims, **characterized in that** the inner annular wall (7) comprises an annular shoulder (7a) and the tubular gas connector (10) comprises an peripheral abutment (12), said peripheral abutment (12) bearing on the annular shoulder (7a), when the tubular gas connector (10) is inserted into the gas inlet (3).

11. Respiratory mask according to any one of the preceding Claims, **characterized in that** a spacing (9) is arranged between the outer annular wall (6) and the inner annular wall (7).

12. Respiratory mask according to any one of the preceding Claims, **characterized in that**, when the tubular gas connector (10) is inserted into the gas inlet (3), a part of the peripheral outer surface of said tubular gas connector (10) delimits with the inner annular wall (7) a gas passage (14), said gas passage (14) being in fluid communication with venting holes (15) arranged in the inner annular wall (7), and with the ambient atmosphere.

13. Respiratory mask according to any one of the preceding Claims, **characterized in that** it further comprises a holding arm (22) projecting upwardly from the hollow body (1), and two lateral arms (21) projecting laterally from the hollow body (1), the two lateral arms (21) and the holding arm (22) being integrally fixed to said hollow body (1), preferably a headgear comprising several straps is fixed to the two lateral arms (21) and the holding arm (22).

14. Respiratory mask according to any one of the preceding Claims, **characterized in that** it further comprises a flexible cushion (26) fixed to the hollow body (1) and comprising a respiratory chamber with an aperture adapted for receiving at least part of the patient's nose, when the patient wears the mask, preferably the flexible cushion (26) comprises one or several membranes.

15. Assembly for delivering a gas to a patient comprising a gas delivery device, such as a medical ventilator, and a respiratory mask according to any one of the preceding Claims.
